Europäisches Patentamt

European Patent Office

Office européen des brevets

(19)

(11) Publication number: **0 154 741 B1**

## EUROPEAN PATENT SPECIFICATION

(12)

(45) Date of publication of patent specification: **03.04.91**  (51) Int. Cl.⁵: **A61L 27/00, C08L 89/06**

(21) Application number: **84307516.9**

(22) Date of filing: **31.10.84**

(54) Clay-collagen complexes and process for their obtention.

(30) Priority: **14.03.84 ES 530599**

(43) Date of publication of application:
**18.09.85 Bulletin 85/38**

(45) Publication of the grant of the patent:
**03.04.91 Bulletin 91/14**

(84) Designated Contracting States:
**AT BE CH DE FR GB IT LI LU NL SE**

(56) References cited:

CHEMICAL ABSTRACTS, vol. 90, no. 23, 4th
June 1979, page 268, abstract no. 182365m,
Columbus, Ohio, US; M. KUBOTA et al.:
"Studies of utilization of collagen. IV. Stud-
ies on insolubilized enzyme using soluble
collagen as the matrix. II. Preparation of
insolubilized enzyme by aggregation of
alpha-amylase, gibbsite, bentonite, and col-
lagen", & HIKAKU KAGAKU 1978, 24(3), 131-9

(73) Proprietor: **TOLSA S.A., INDUSTRIAL
Nunez de Balboa 51
28001 Madrid(ES)**

(72) Inventor: **Berenguer, Antonio Alvarez
C/Fermin Caballero 58
28034 Madrid(ES)**
Inventor: **Franco, Jose Gavilanes
C/Juan Bravo 55-4 Izquierda
28006 Madrid(ES)**
Inventor: **Del Pozo, Alvaro Martinez
C/Santa Engracia 21-2 Derecha
28010 Madrid(ES)**
Inventor: **Perez, Nieves Olmo
C/Azuago 20-2 Izquierda
28039 Madrid(ES)**
Inventor: **Iracheta, Lizarbe Maria-Antonia
C/Almansa 88-4C
28040 Madrid(ES)**
Inventor: **Castells, Rafael Perez
C/Dr. Drumen 6-3 Izquierda
28012 Madrid(ES)**

(74) Representative: **Pearce, Anthony Richmond et
al
MARKS & CLERK Alpha Tower Suffolk Street
Queensway Birmingham B1 1TT(GB)**

## Description

For a material to be used, with clinical purposes, in the manufacture of various types of prosthesis, it must have certain properties, since without them the material would not be useful therefor. Amongst such properties, the possibility of showing cellular adherence but not repulsion phenomena at an immunological level should be emphasised. Thus, clays and specifically those pertaining to the Sepiolite-Paligorskite group, are not described as having a capacity for stimulating lymphocytes. Furthermore, collagen is the main protein, for various reasons, in the extracellular matrix assembly and, consequently, in all kinds of cellular contacting phenomena.

Chemical Abstracts Vo. 90, No. 23, page 268, Abstract No. 182365m discloses the preparation of insolubilized enzyme by adsorbing $\alpha$-amylase on gibbsite, and then ading a bentonite suspension and acid soluble collagen, followed by neutralization with NaOH, to produce a neutralized flock. After filtering and drying, the dried flocks are treated with glutaraldehyde to endow practical usability.

The present invention relates to novel clay and collagen complexes and to a process for their preparation, so that the resultant structure is an immunologically inert material capable, on its own or acting as a support for others, of carrying out biomaterial functions.

According to the present invention, there is provided a clay-collagen complex comprising a clay having a particle size of lower than 44 $\mu$m and a modified or unmodified collagen of any of types I to VII in a stoichiometric ratio of 100/1-80, characterized in that the clay is selected from Attapulgite and Sepiolite. The collagen may be any of the different varieties corresponding to each of the above specified types, resulting from normal purification processes or any kind of chemical or enzymatic modification process. The complex does not behave as a mere mixture, since in aqueous dispersions it does not release collagen molecules, not even when it is subjected to an ultrasonic treatment with frequencies of 6 microns (peak to peak) for 1 minute.

The type of structure of the complex is not random, but the collagen and sepiolite fibers are arranged parallely.

The process of the invention is based on utilizing the repetitive structure presented by the clay as an inductor support for the deposit and subsequent aggregation of collagen molecules, under conditions in which the protein exhibits its highest solubility. The fact that the molecules of this protein present a characteristic and constant surface load distribution helps to favour the formation of the above-mentioned complexes.

The polypeptide component of the complex, that is the collagen, is a protein comprised of three subunits having a total molecular weight of about 30,000 D. The most characteristic feature of this protein is its peculiar rod-shaped three-dimensional structure formed of a triple helix in which each of the three components, in turn, is present in a Poly-Pro II helicoidal form. The function of the collagen can refer to structural aspects derived from its triple helix characteristic. It carries out support functions, facilitating the molecular linkage on which the cellular growth takes place. Thus, it is the essential polypeptide component in connective tissue. However, the term "collagen" does not refer to a single protein, but to a family thereof, the members of which, although having the basic feature of the triple helix, possess some structural differences. Thus, the different types of collagen occur. The most abundant is type I which has been widely studied and is commonly known. It occurs in the skin, tendons, bone and dentin. It can respond to two different types of associations having three subunits each: $[\alpha_1(I)_2\alpha_2(I)]$ and $[\alpha_1(I)]_3$, this latter variety only being observable in the skin. Type II collagen occurs in the cartilages and invertebral disks, the stoichiometry of which is $\alpha(II)_3$. Likewise, other forms of collagen occur in the cartilage, such as variety $(1\alpha2\alpha3\alpha)$ and HMW and LMW; these two latter correspond to the stoichiometry $(C2)_2$ C3 and $(LMW)_3$, respectively. Type III, $\alpha(III)_3$ collagen is characteristic of fetal tissues. Type IV $[\alpha_1(IV)_2 \alpha_2(IV)]$ as well as type V occur in basal membranes. This latter type of collagen also occurs in the muscle, placenta and uterus and has, in turn, three varieties: $\alpha_1(V)_3, \alpha_1(V)[\alpha_2(V)]_2, \alpha_1(V)\alpha_2(V)\alpha_3(V)$. There are also other types of collagen, which are a minority and have recently been discovered. Type VI, VII and VIII collagens could be classified in this type. These three types occur in the placenta, skin and intima of the aorta and the stoichiometries thereof are $(SC2)_2$ SC3 or basic$_2$-acid. Type VII collagen occurs in the corium and is $(LC)_3$, while type VIII has been observed in the aorta endothelial cellular cultures and there is no data concern ing its stoichiometry. The various types of subunits already mentioned, differ in their aminoacid sequence, in the number of hydroxylysine moities, as well as in their molecular weight and number of glycolysed hydroxylysine moieties.

The various types of collagen also differ in their functional behaviour, which differences arise from the described structural variability. Hence, type I, II and III collagens are capable of forming microfibrilla in thermodependent aggregation processes. Type IV collagen gives rise to net-like aggregates, with inter-

2

molecular contact points in which 4 molecules coexist, 4 triple helixes, such coexisting fragments being denominated as collagen 75. Type VI collagen is formed of peculiar fibrous structures comprised of 8 triple helixed units, with zones at which 4 triple helixes interact and zones at which only 2 interact; in any case, the structure of the assembly is also superhelicoidal having triple helixes. Type VII, longchain collagen is longer in its structural unit, emphasising the presence of zones having disulfide bridges in the terminal ends NH2 and -COOH. Referring to type VII collagen, as already mentioned, no prognostications can be made concerning its molecular aggregates.

Independently of the mentioned structural differences and the formal differences between aggregates, it could be stated that collagen is a protein whose purpose is that of forming the extracellular matrix, the specific requirements thereof in the different tissues conditioning the presence of one type of collagen or the other. This function is precisely carried out through the different types of molecular aggregates.

The second component of the described complexes is clay. Attapulgia and Sepiolite pertain to this group of Sepiolite-Paligorskite clays. They are fibrous type hydrated aluminum magnesium silicates. Sepiolite is the magnesium variety of the group. The structure of both clays consists of double $SiO_2$ chains extending parallel to the axis C and joined together through the oxygens of their longitudinal borders. The vertices of the tetrahedrons in adjacent chains point in opposite directions. The $SiO_2$ layers thus formed are placed on one another so that the vertices of the tetrahedrons point inwards of each two consecutive layers and are joined together by a discontinuous aluminum magnesium layer in octahedral coordination. The octahedral layer is completed with hydroxyl groups in the centre and $OH_2$ groups at the sides.

The structure is represented in the form of a table in which there are longitudinal channels which at the front resemble a brick wall from which bricks have been removed alternately.

Sepiolite and Attapulgite differ in the dimensions of the layers and in the composition of the layer arranged in octahedral coordination. Thus, the Sepiolite octahedral layer is magnesium, while that of Attapulgite is aluminum-magnesium. The dimensions of the cell unit are of 12.9 Å x 18.0 Å and of 13.4 Å x 26.9 Å x 5.28 Å for Attapulgite and Sepiolite, respectively. Therefore, the zeolitic channels of the Sepiolite are larger than those of Attapulgite.

Another important difference is the various abundances of silanoids on the surface of the Attapulgite and Sepiolite fibers. Thus, the surface of the Sepiolite particles has an SiOH group each 5 Å, while this amount is slightly lower on that of Attapulgite. This is a very important feature, since these groups play an important role in the interaction will collagen molecules.

In the specific case of sepiolite-collagen complexes, highly pure (>80%) wet or dry micronised sepiolite is used, although use of the former is preferred.

To carry out this invention, the granulometry and the shape of the micronised particles are also important. Products having a particle size lower than 44 μm have been used, although those products having a size lower than 10 μm are preferred. Although micronised products in which the particle is formed of a group of degraded fibers can be used, the best results are obtained with those micronized products conserving the fiber intact.

According to the process of the present invention, the clay-collagen complexes are formed when both components are put in contact in a suitably sized container. This process is conducted at a temperature of from 4 to 60° C, preferably from 15 to 40° C. The pH of the interchanging medium must be lower than 9.0, preferably between 2.5 and 4.0. The formation kinetics of the complexes has a high initial speed, so that the final results are not appreciably dependent on the time in the range of from 1 to 60 minutes. However, periods of time of from 15 to 25 minutes give very satisfactory results.

To prepare the mentioned complexes, the re action mixtures must have the following stoichiometry:

clay      100
collagen   1-80

the proportion of the second component being, preferably, of from 20 to 40. The clay-collagen complexes, thus formed, can be separated, either by centrifugation or by decantation, without modifying the product resulting from the formation process.

The invention is illustrated by the following examples.

EXAMPLE 1

2 mg/ml of type I collagen were dissolved in water with the pH adjusted to 3.6 with HCl. 3 ml of this solution were added to 10 mg of intact fiber micronized Sepiolite. It was vigorously stirred for 30 minutes and incubated at 26° C for 20 minutes. The complex was removed by centrifugation to 100 g for 5 minutes. The complex was stable, moldable, and acquired resistance and strength upon drying.

EXAMPLE 2

Collagen absorption in intact fiber micronized Sepiolite.
The results of the formation of the clay-collagen complexes are present in figures 1 and 2.

Fig. 1

Fig. 2

——————— collagen in the form of a complex

_____ collagen in the form of solution

Figure 1 represents the type I collagen retention in the concentration range of from 0.1 to 1.6 mg/ml, for a 10 mg clay mass. The modification of the properties of monomer collagen is presented in the collagen fluorescence spectrum, in the form of a complex, in comparison with that of isolated collagen, both for an excitation wave length of 276 nm; both are presented in figure 2.

These complexes can be used as a cellular culture support, or as a base for the manufacture of artificial skins and bones.

EXAMPLE 3

Collagen abosrption in degraded fiber micronized Sepiolite.

Figure 3 represents the retention graph of this type of clay for type I collagen, with a concentration range of 0.1 mg/ml of protein.

Fig. 3

In this case, the sepiolite can interact with a lesser amount of type I collagen, although the protein level absorbed or supported on micronized sepiolite with degraded fiber is lower than in the event intact fibers are used, this material is also suitable for obtaining collagen sepiolite complexes.

**Claims**

1. A clay-collagen complex comprising a clay having a particle size of lower than 44 μm and a modified or unmodified collagen of any of types I to VII in a clay/collagen stoichiometric ratio of 100/1-80, characterized in that the clay is selected from Attapulgite and Sepiolite.

2. A clay-collagen complex according to claim 1, characterized in that the clay has a particle size lower than 10μm or it is micronized so that the particles are formed of groups of degraded fibers.

3. A clay-collagen complex according to claim 1, characterized in that the clay has a particle size lower than 10μm or it is micronized so that the particles are intact fibers.

4. A process for producing a clay-collagen complex which is as claimed in any preceding claim, said process comprising the step of reacting the collagen with the clay at a temperature of from 4 to 60°C, a pH lower than 9.0 and for 1 to 60 minutes in a reaction mixture wherein the clay/collagen stoichiometry is 100/1-80.

5. A process as claimed in claim 4, characterized in that the reacting step is carried out at a temperature of from 15 to 40°C.

6. A process as claimed in claim 4 or 5, characterized in that the reacting step is carried out at a pH of from 2.5 to 4.0

7. A process as claimed in claim 4, 5 or 6, characterized in that the reaction step is carried out for a period of time of from 15 to 25 minutes.

8. A process as claimed in claim 4, 5, 6 or 7, characterized in that the reaction medium is an aqueous medium.

**Revendications**

1. Complexe d'argile-collagène comprenant une argile ayant une taille de particule inférieure à 44 μm et un collagène modifié ou non modifié de l'un des types I à VII dans un rapport stoechiométrique argile/collagène de 100/1-80, caractérisé en ce que l'argile est choisie parmi l'attapulgite et la sépiolite.

2. Complexe d'argile-collagène suivant la revendication 1, caractérisé en ce que l'argile a une taille de particule inférieure à 10 μm ou bien elle est micronisée de telle sorte que les particules soient formées de groupes de fibres dégradées.

3. Complexe d'argile-collagène suivant la revendication 1, caractérisé en ce que l'argile a une taille de particule inférieure à 10 μm ou bien elle est micronisée de telle sorte que les particules soient des fibres intactes.

4. Procédé de production d'un complexe d'argile-collagène tel que revendiqué suivant l'une quelconque des revendications précédentes, ce procédé comprenant l'étape de mise en réaction du collagène avec l'argile à une température de 4 à 60° C, un pH inférieur à 9,0 et pendant 1 à 60 minutes dans un mélange de réaction dans lequel la stoechiométrie argile/collagène est de 100/1-80.

5. Procédé suivant la revendication 4, caractérisé en ce que l'étape de réaction est réalisée à une température de 15 à 40° C.

6. Procédé suivant l'une ou l'autre des revendications 4 et 5, caractérisé en ce que l'étape de réaction est réalisée à un pH de 2,5 à 4,0.

7. Procédé suivant l'une quelconque des revendications 4, 5 et 6, caractérisé en ce que l'étape de réaction est réalisée pendant une période de temps de 15 à 25 minutes.

8. Procédé suivant l'une quelconque des revendications 4, 5, 6 et 7, caractérisé en ce que le milieu de réaction est un milieu aqueux.

**Ansprüche**

1. Ton-Kollagen-Komplex umfassend einen Ton mit einer Teilchengröße von unter 44 μm und ein modifiziertes oder unmodifiziertes Kollagen einer der Typen I bis VII in einem stöchiometischen Ton/Kollagen-Verhältnis von 100/1-80, dadurch gekennzeichnet, daß der Ton aus Attapulgit und Sepiolith ausgewählt ist.

2. Ton-Kollagen-Komplex nach Anspruch 1, dadurch gekennzeichnet, daß der Ton eine Teilchengröße von unter 10μm hat oder so pulverisiert ist, daß die Teilchen von Gruppen abgebauter Fasern gebildet sind.

3. Ton-Kollagen-Komplex nach Anspruch 1, dadurch gekennzeichnet, daß der Ton eine Teilchengröße von unter 10μm hat oder so pulverisiert ist, daß die Teilchen intakte Fasern sind.

4. Verfahren zur Herstellung eines in einem vorhergehenden Anspruch beanspruchten Ton-Kollagen-Komplexes, welches verfahren den Schritt der Umsetzung des Kollagens mit dem Ton bei einer Temperatur von 4 bis 60° C, einem pH-Wert von unter 9,0 und während einer Zeitdauer von 1 bis 60 Minuten in einer Reaktionsmischung, in welcher die Ton/Kollagen-Stöchiometrie 100/1-80 beträgt, umfaßt.

5. Verfahren, wie in Anspruch 4 beansprucht, dadurch gekennzeichnet, daß der Schritt der Umsetzung bei einer Temperatur von 15 bis 40 °C durchgeführt wird.

6. Verfahren, wie in Anspruch 4 oder 5 beansprucht, dadurch gekennzeichnet, daß der Schritt der Umsetzung bei einem pH-Wert von 2,5 bis 4,0 durchgeführt wird.

7. Verfahren, wie in Anspruch 4, 5 oder 6 beansprucht, dadurch gekennzeichnet, daß der Schritt der Umsetzung während einer Zeitdauer von 15 bis 25 Minuten durchgeführt wird.

8. Verfahren, wie in Anspruch 4, 5, 6 oder 7 beansprucht, dadurch gekennzeichnet, daß das Reaktionsmedium ein wässeriges Medium ist.